(19) Europäisches Patentamt
European Patent Office
Office européen des brevets

(11) Publication number: **0 446 720 A2**

(12) **EUROPEAN PATENT APPLICATION**

(21) Application number: **91103076.5**

(22) Date of filing: **01.03.91**

(51) Int. Cl.5: **C07C 43/29**, C07C 317/22, C07C 323/20, C07C 49/813, C07C 41/22, C07C 45/63, C07C 315/04, C07C 319/20

(30) Priority: **08.03.90 IL 93684**
**20.02.91 IL 97300**

(43) Date of publication of application:
**18.09.91 Bulletin 91/38**

(84) Designated Contracting States:
**BE CH DE ES FR GB IT LI NL**

(71) Applicant: **Bromine Compounds Ltd.**
**Makleff House P.O.B. 180**
**Beer-Sheva 84101(IL)**

(72) Inventor: **Oren, Jacob**
**8/9, Reuven Street**
**Qiryat Bialik(IL)**
Inventor: **Hermolin, Joshua**
**14, Ha'Avoda Street**
**Ramat-Hasharon 47445(IL)**
Inventor: **Feldman, David**
**18, Peretz Markish Street**
**Haifa(IL)**
Inventor: **Zviely, Michael**
**3, Haim-Hazaz Street**
**Savioney-Dania, Haifa 34984(IL)**
Inventor: **Hacham, Ronny**
**11, Haneviem Street**
**Holon(IL)**

(74) Representative: **Perani, Aurelio et al**
**c/o JACOBACCI-CASETTA & PERANI Via Visconti di Modrone 7**
**I-20122 Milano(IT)**

(54) **Novel brominated phenoxy compounds and processes for their preparation.**

(57) Compounds of the formula

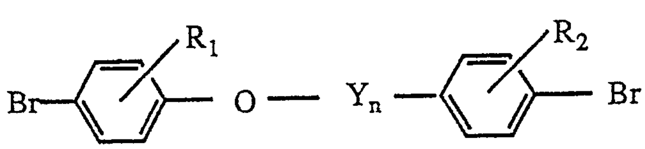

(I)

wherein:

n = 0 or 1;

$R_1$ and $R_2$ are $C_1$ to $C_3$ alkyl groups, or $R_2$ may be H when n = 0;

Y is selected from the group:

or

EP 0 446 720 A2

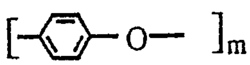 ; 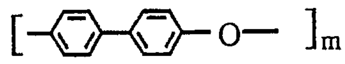

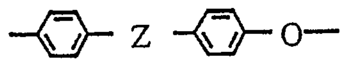

wherein:

m = 1-6;

Z = $SO_2$,S,C=O,$CH_2$ or $C(CH_3)_2$;

and a process for their preparation are described.

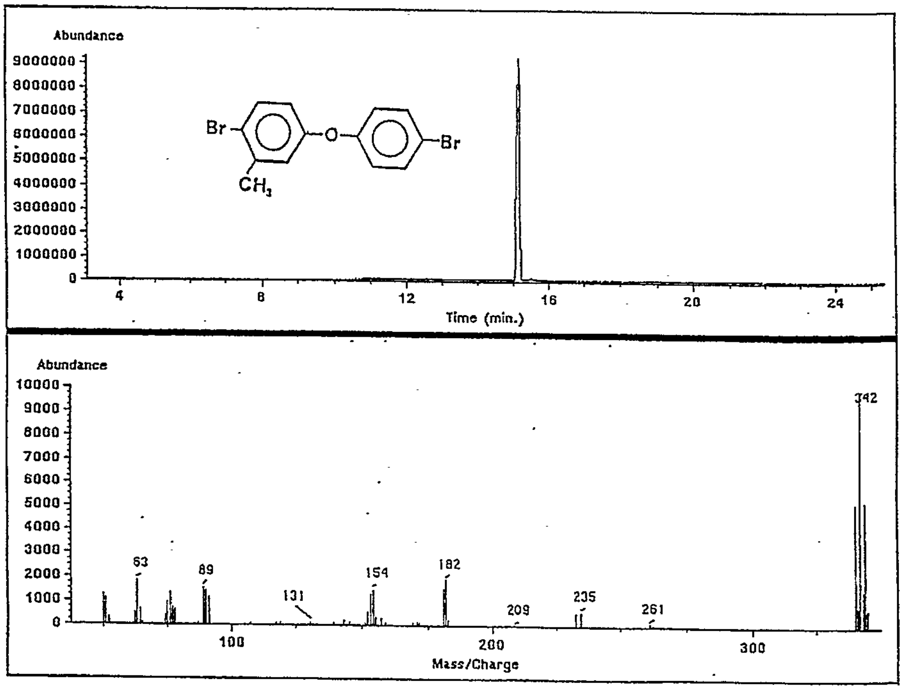


Fig. 1

## Field of The Invention

The present invention relates to novel brominated phenoxy compounds and to processes for their preparation.

## Summary of The Invention

The compounds of the invention have the formula:

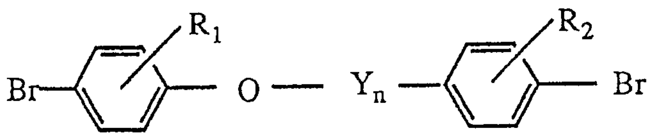

(I)

wherein:

n = 0 or 1;

$R_1$ and $R_2$ are $C_1$ to $C_3$ alkyl groups, or $R_2$ may be H when n = 0;

Y is selected from the group:

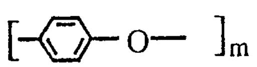

;

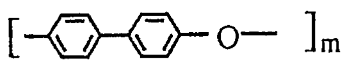

or

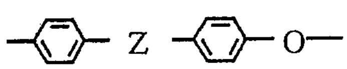

wherein:

m = 1-6;

Z = $SO_2$,S,C = O,$CH_2$ or $C(CH_3)_2$;

with the proviso that $R_1$ and $R_2$ cannot both be $CH_3$ when n = 0.

The compounds of the invention can be used as precursors of monomers and polymers, and include the following illustrative and non-limitative compounds:

4,4'-Dibromo-3-methyl-diphenylether
4,4'-Dibromo-2-methyldiphenyl ether
4,4'-Dibromo-2-isopropyldiphenyl ether
1,4-Bis (4'-bromo-3'-methylphenoxy) benzene
1,4-Bis (4'-bromo-2'-methylphenoxy) benzene
4,4'-Bis (4"-bromo-2"-methylphenoxy) biphenyl
4,4'-Bis (4"-bromo-3"-methylphenoxy) biphenyl
4,4'-Bis (4"-bromo-2"-methylphenoxy) diphenyl ether
4,4'-Bis (4"-bromo-3"-methylphenoxy) diphenyl ether
4,4'-Bis (4"-bromo-2"-methylphenoxy) diphenyl sulfone
4,4'-Bis (4"-bromo-3"-methylphenoxy) diphenyl sulfone
4,4'-Bis (4"-bromo-2"-methylphenoxy) diphenylmethane
4,4'-Bis (4"-bromo-3"-methylphenoxy) diphenylmethane
4,4'-Bis (4"-bromo-2"-methylphenoxy) diphenyl sulfide
4,4'-Bis (4"-bromo-3"-methylphenoxy) diphenyl sulfide
2,2'-Bis [4'(4"-bromo-2"-methylphenoxy) phenyl] propane
2,2'-Bis [4'(4"-bromo-3"-methylphenoxy) phenyl] propane
4,4'-Bis (4"-bromo-2"-methylphenoxy) benzophenone

4,4'-Bis (4''-bromo-3''-methylphenoxy) benzophenone

These novel compounds are useful for the preparation of a variety of 4,4'-disubstituted di- and polyphenyl compounds. These latter compounds, some of which are also novel and which are described in copending patent applications of the same applicant, include, e.g., 4,4'-dihydroxy-3-methyl-diphenylether, 4,4'-diamino-3-methyl-diphenylether, 4,4'-diamino-3-methyl-diphenylether-bis-maleimide and similar compounds.

These 4,4'-disubstituted phenyl ethers are industrially useful monomers for the production of high performance polymers for application in the aviation, electrical, electronics and aerospace industries. Because of the many applications, a wide range of properties are sought by structural changes in the monomers. Thus, altering these monomers by substituents in the aromatic ring, and/or the substitution of one radical which serves to bind two of the aromatic rings by another, affects the softening point, flexibility, heat resistance, etc., of the resins produced therefrom. The improved properties they provide can benefit a wider range of applications which seek better performance from lighter-weight materials. The family of compounds described herein serve to provide this range of variability in the polymer properties.

The preparation of 4,4'-dibromo-3-methyl-diphenylether (DBMDPE) is representative of the preparation methods of the compounds of the invention, with the required changes in, each case, according to the starting material and the required product, and therefore will be discussed at some length hereinafter.

This compound can be prepared by reacting m-phenoxytoluene (m-PHT) with bromine or other brominating agent in substantially complete darkness, to avoid the undesired bromination of the methyl group. When the reaction is carried out under these conditions DBMDPE is formed in high yield and with high purity.

## Detailed Description of the Invention

In general, the process of the invention comprises reacting a compound of formula

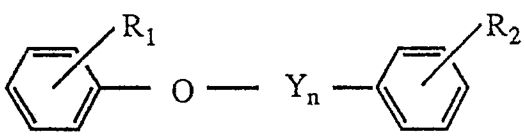

(II)

wherein $R_1$, $R_2$, Y and n have the meanings hereinbefore defined, with a brominating agent in the substantial absence of light.

Any suitable bromination agent can be used in the bromination reaction, as long as it is compatible with the reaction mixture. Preferred brominating agents include e.g., $Br_2$ or dibromodimethylhydantoin, or bromine may be generated in situ , e.g. by employing HBr together with an oxidizing agent, such as $H_2O_2$ or $NaBrO_3$, or the like known oxidizing agents. As will be appreciated by the skilled chemist, oxidizing agents can also be used together with $Br_2$ to enhance the bromination reaction by generating free bromine from HBr generated during the reaction.

The reaction can be carried out in a solvent, or without a solvent, but the presence or absence of such a solvent may affect the selectivity of the bromination. When a solvent is employed, it is preferred to employ a solvent which is unreactive under the reaction conditions, such as halocarbons, e.g. dichloromethane, chlorobenzene and 1,2-dichloroethane or acetonitrile.

The reaction temperature also affects the selectivity of the bromination as well as the reaction rate. The higher the temperature the lower is the selectivity, but the faster is the reaction rate. Therefore, reaction temperatures may vary, depending on the purity of the desired product as well as other economic factors. It is preferred to add the brominating agent at a temperature in the range of about -10° to about 25° C, and to carry out the reaction at a temperature of about -10° to about 90° C. However, higher or lower temperatures can be employed, as long as they are compatible with the solvent employed and do not adversely affect the reactants or the product.

The characterizing data and the method of preparation will be illustrated by the following examples, which are not to be a construed as a limitation of the invention. Analytical data reported in the examples were obtained with the instruments and conditions, generally as described below.

GC

Gas - chromatograph - Varian 3400
Oven: Initial temperature 100° C, held 1 min., then raised to 250° C at 15°/min.

| | |
|---|---|
| Injector: | 250° C |
| Detector (transfer line): | 300° C |
| Column: | HP-1 (100% methyl polysiloxane), 5 m x 0.53 mm (megabore). |
| Injection amounts: | 1 μl |
| Flow: | 13 ml/min. |
| Retention Time: | 7.05 min. |

GC/MS

Gas-chromatograph HP 5890 A
Oven: Initial temperature 100° C, held 1 min. then raised to 240° C at 15°/min.

| | |
|---|---|
| Injector: | 230° C. |
| Detector (transfer line): | 250° C |

Column SUPELCO (fused silica capillary column) 30m x 0.25 mm.

| | |
|---|---|
| Split ratio: | 1:50 |
| Injection amounts: | 1 μl |
| Flow: | 0.6 ml/min. |
| Retention time: | 15.2 min. |

Mass Spectrometer HP 5970

| | |
|---|---|
| Range: | 40-550 a.m.n |
| Scan: | every 0.9 second. |

NMR spectra: Bruker WP 200 MHz

| | $^{1}H$-NMR | $^{13}C$-NMR |
|---|---|---|
| Solvent: | $CDCl_3$ | DMSO - $d_6$ |
| Scans: | 50 | 10,000 |
| Reference: | TMS | TMS |

IR: FTIR Nicolet 5 MX

| | |
|---|---|
| Range: | 400-4600 $cm^{-1}$ |
| Scan: | 10 (every 1.0 second.) |
| Sample: | 0.8 mg/80 mg KBr |

**Brief Description of the Drawings**

- Fig. 1 is the mass spectra of DBMDPE;
- Fig. 2 is the $^{1}H$-NMR spectrum of DBMDPE in $CDCl_3$;
- Fig. 3 is the $^{13}C$-NMR spectrum of DBMDPE in DMSO-$d_6$;
- Fig. 4 is the IR spectrum of DBMDPE;
- Fig. 5 is the GCMS spectrum of 4,4'-dibromo-2-isopropyldiphenyl ether (DBIPE);

- Fig. 6 is the NMR spectrum of DBIPE;
- Fig. 7 is the mass spectrum of 1,4-bis (4'-bromo-3'-methylphenoxy) benzene (BB3MPZ);
- Fig. 8 is the NMR spectrum of BB3MPZ;
- Fig. 9 is the mass spectrum of 1,4-bis (4'-bromo-2'-methylphenoxy) benzene (BB2MPZ);
- Fig. 10 is the mass spectrum of 4,4'-bis (4"-bromo-2"-methylphenoxy) biphenyl (BBMPL);
- Fig. 11 is the mass spectrum of 4,4'-bis (4"-bromo-2"-methylphenoxy) diphenyl sulfone (BBMPS);
- Fig. 12 is the NMR spectrum of BBMPS;
- Fig. 13 is the mass spectrum of 4,4'-dibromo-2-methyldiphenyl ether (DBMPE);
- Fig. 14 is the NMR spectrum of DBMPE; and
- Fig. 15 is the mass spectrum of 4,4'-bis(4"-bromo-2"-methylphenoxy) diphenyl ether (BBMPE).

**Example 1**

**Bromination of m-PHT in dichloromethane**

To a four-necked round-bottomed flask equipped with a stirrer, a condenser, a dropping funnel and a thermometer, containing a stirred solution of m-PHT (460 g, 2.5 moles) in one liter of dichloromethane, there were added 840 g ( 5.25 moles) of $Br_2$. The top of the condenser was equipped with a trap to absorb HBr released during the reaction. $Br_2$ was added at a temperature between -5° and 0° C and in the dark during one hour, and after addition, the reaction mixture was stirred for an additional 1 hour at about 25° C. The progress of the reaction was determined by GC. Excess bromine and traces of HBr were neutralized with aqueous 10% $NH_3$ (150 ml). Two phases formed in the reaction, which were separated, and the organic layer was washed with water (200 ml). After distillation of the solvent in the organic phase, DBMDPE (820 g) was obtained, containing 97% of the desired isomer (determined by GC) which represents a yield of 93%.

After crystallization from methanol, a product of 99% purity was obtained, containing 46.4% Br, and having a melting point of 44-46° C. The crystallization from ethanol gave identical results.

The product so obtained is characterized by the following spectral data:

GC under the conditions described above shows a main peak (99.6%) at a retention time of 7.05 min.

Fig. 1 shows the mass spectra of the product;
Fig. 2 shows the $^{1}$H-NMR spectrum of the product, in which $CDCl_3$ is used as a solvent;
Fig. 3 is the $^{13}$C-NMR spectrum of the product obtained in DMSO-$d_6$; and
Fig. 4 is the IR spectrum of the product.

**Example 2**

**Bromination of m-PHT in 1,2-dichloroethane**

Example 1 was repeated but using 1,2-dichloroethane as the solvent instead of dichloromethane. Substantially identical results were obtained.

**Example 3**

**Bromination of m-PHT using $H_2O_2$**

Using the same arrangement as in Example 1, to a mixture of m-PHT (184 g, 1.0 mole) in 500 ml of 1,2-dichloroethane and 200 ml of water, kept at a temperature of about 0°-5° C in substantially complete darkness, there were added 176 g (1.1 moles) of $Br_2$. The addition was carried out during about 30 minutes, after which time 30% $H_2O_2$ (131 g, 1.15 moles) was added at 50° C over 1 hour. The progress of the reaction and its completion were monitored using GC.

After about 2 hours, full conversion was obtained, and the phases were separated. After removal of the solvent from the organic phase, crude DBMDPE (335 g) was obtained. The content of the desired isomer in the crude product was 95%, as determined by GC. A pure product was obtained by crystallization as in Example I.

**Example 4**

**Bromination of m-PHT using $NaBrO_3$**

Using the same setup as in the previous examples, a mixture of 184 g (1.0 mole) of m-PHT in 1,2-dichloroethane (500 ml), 55 g (0.36 moles) of $NaBrO_3$ and 200 ml of water, at a temperature of 25° C and in the dark, was reacted with 176 g (1.1 moles) of $Br_2$. The addition of $Br_2$ to the reaction mixture was carried out during about 30 minutes. The progress of the reaction and its completion were monitored and determined by GC.

Full conversion was obtained in about 2 hours, after which time the phases were separated. After removal of the solvent from the organic phase, DBMDPE (340 g) was obtained. The content of the desired isomer in the crude product was 97%, as determined by GC. A pure product was obtained by crystallization as in the previous examples.

**Example 5**

**Bromination of m-PHT without a solvent**

Using the same reaction vessel as in the previous examples, 460 g (2.5 moles) of m-PHT, at 5° C and in complete darkness, were reacted with $Br_2$. $Br_2$ (832 g, 5.2 moles) was added under mechanical stirring and the temperature of the reaction mixture raised. Before the end of the reaction, after 90% of the $Br_2$ was added, the temperature of the reaction mixture reached 45° C. After addition of the total $Br_2$ the reaction mixture was stirred for 1 hour. The progress of the reaction was followed by GC.

Full conversion was determined after about 2 hours, at which time excess $Br_2$ and traces of HBr were neutralized with aqueous 10% NH3 (about 150 ml). Separation of the organic phase at about 50° C gave crude DBMDPE (840 g). The desired isomer in the crude product was about 89%, as determined by qualitative GC.

**Example 6**

**Bromination of m-PHT using DBDMH**

Using the same reaction setup as in the previous examples, a suspension of m-PHT (36.8 g, 0.2 moles) and dibromodimethylhydantoin (62.9 g, 0.22 moles) in acetonitrile (120 ml) was maintained under reflux. The reaction was monitored by GC. A 40% conversion to 4,4'-dibromo-3-methyl-diphenylether was observed after 3 hours, and a conversion of 95% after 10 hours.

**Example 7**

Example 1 was repeated using only 3.1 moles of $Br_2$ instead of 5.25 moles. A 1:4 mixture of 4,4'-dibromo-3-methyl-diphenylether and 4-bromo-3-methyl-diphenylether was obtained.

As will be apparent to a skilled chemist, because the first step (monobromination) in the reaction is much faster than the second bromination, it is possible to isolate the mono-brominated product. Likewise, it is possible to employ mono-brominated m-PHT as the starting material, instead of m-PHT. Thus, if mono-brominated m-PHT is available from any source, it is possible to brominate it to obtain DBMDPE, and such a process is also intended to form part of the invention.

**Example 8**

**Preparation of 4,4'-dibromo-2-methyldiphenyl ether (DBMPE)**

Bromine (168 g, 1.05 moles) was added to a stirred solution of 2-phenoxytoluene (92 g, 0.5 moles) in dichloromethane (368 g) in a five-necked 1 liter round-bottomed flask equipped with a stirrer, a condenser, a dropping funnel and a thermometer. To the top of the condenser was attached a trap to absorb hydrobromic acid released during the reaction. The addition of the bromine at a temperature in the range between 3-7° C and in the dark took one hour, after which the reaction mixture was stirred for a further hour. The progress of the reaction and its completion were determined by GC. Excess bromine and traces of hydrobromic acid were neutralized with aqueous 25% $NH_3$ (48 ml). The phases were separated and the organic layer washed twice with water (100 ml). After distillation of the solvent in the organic phase, DBMPE was obtained, containing 99.3% of the desired isomer (determined by qualitative GC).

After fractional distillation, 152.4 g of DBMPE in 99.5% purity was obtained. Chemical yield: 87.7%, m.p. 33° C, 46.4% Br (calc'd, 46.8%). The mass spectrum and NMR of the product are shown in Figs. 13

and 14, respectively.

**Example 9**

**Preparation of 4,4'-dibromo-2-isopropyldiphenyl ether (DBIPE)** Bromine (16.8 g, 0.105 moles) was added to a stirred solution of 2-isopropyldiphenyl ether (10.6 g, 0.05 moles) in dichloromethane (42.5 g) in a three-necked 100 ml round-bottomed flask equipped with a stirrer, a condenser, a dropping funnel and a thermometer. To the top of the condenser were attached two consecutive 20% NaOH traps to absorb hydrobromic acid released during the reaction. The addition of the bromine at a temperature in the range between 2-5°C and in complete darkness took one and a half hours, after which the reaction mixture was stirred for a further half-hour. The progress of the reaction and its completion were determined by GC. Excess bromine and traces of hydrobromic acid were neutralized with aqueous 10% $NH_3$ (60 ml). The phases were separated and the organic layer washed twice with water (50 ml). After distillation of the solvent in the organic phase, crude DBIPE was obtained, containing 93.6% of the desired product in ca. 90% yield (determined by qualitative GC). GCMS analysis of the purified product produced the spectrum of Fig. 5. Its NMR spectrum is given in Fig. 6.

**Example 10**

**Preparation of 1,4-bis (4'-bromo-3'-methylphenoxy) benzene (BB3MPZ)**

Bromine (108.7 g, 0.34 moles) was added to a stirred solution of 1,4-bis (3'-methylphenoxy) benzene (98.5 g, 0.34 moles) in dichloromethane (400 g) in a four-necked round-bottomed flask equipped with a stirrer, a condenser, a dropping funnel and a thermometer. To the top of the condenser was attached a trap to absorb hydrobromic acid released during the reaction. The addition of the bromine at a temperature in the range between 0-2°C and in complete darkness took one hour, after which the reaction mixture was stirred for a further hour at room temperature (~25°C). The progress of the reaction and its completion were determined by GC. Excess bromine and traces of hydrobromic acid were neutralized with aqueous 10% $NH_3$ (50 ml). The phases were separated and the organic layer washed with water (60 ml). After distillation of the solvent in the organic phase, crude BB3MPZ (148.5 g) was obtained, containing 97% of the desired isomer (determined by qualitative GC) which represents a yield of 94.6%.

After crystallization (from methanol or ethanol), a product of >99% purity was obtained (35.7% Br, m.p. 110-113°C). Its mass spectrum and NMR spectrum are shown in Figs. 7 and 8, respectively.

**Example 11**

**Preparation of 1,4-bis (4'-bromo-2'-methylphenoxy)benzene (BB2MPZ)**

Bromine (67.2 g, 0.42 moles) was added to a stirred solution of 1,4-bis (2'-methylphenoxy) benzene (58 g, 0.2 moles) in dichloromethane (232 g) in a 1 liter five-necked round-bottomed flask equipped with a stirrer, a condenser, a dropping funnel and a thermometer. To the top of the condenser were attached two consecutive 20% NaOH traps to absorb hydrobromic acid released during the reaction. The addition of the bromine at a temperature in the range between 0-5°C and in the dark took two hours, after which the reaction mixture was stirred for a further hour. The progress of the reaction and its completion were determined by GC. Excess bromine and traces of hydrobromic acid were neutralized with aqueous 12.5% $NH_3$ (100 ml). The phases were separated and the organic layer washed twice with water (50 ml). After distillation of the solvent in the organic phase, crude BB2MPZ (83.4 g) was obtained, containing 98.5% of the desired product (determined by qualitative GC), which represents a yield of 92.8% and selectivity of 99.7%. After crystallization, a product of >99% purity was obtained (35.3% Br, calc'd. 35.7% Br, m.p. 103.8°C). Its mass spectrogram is given in Fig. 9.

**Example 12**

**Preparation of 4,4'-bis(4"-bromo-2"-methylphenoxy)biphenyl (BBMPL)**

Bromine (13.4 g, 0.084 moles) was added to a stirred solution of 4,4'-bis (2'-methylphenoxy) biphenyl (14.6 g, 0.04 moles) in dichloromethane (58 g) in a 100 ml three-necked round-bottomed flask equipped with a stirrer, a condenser, a dropping funnel and a thermometer. To the top of the condenser were

attached two subsequent 20% NaOH traps to absorb hydrobromic acid released during the reaction. The addition of the bromine at a temperature in the range 0-10 °C and in complete darkness took 80 minutes.

The progress of the reaction and its completion were determined by GC. Excess bromine and traces of hydrobromic acid were neutralized with aqueous 25% $NH_3$ (7 ml). The phases were separated and the organic layer washed with water (3 x 45 ml).

After distillation of the solvent in the organic phase, crude BBMPL was obtained (20.8 gr), containing 92.4% of the desired isomer (determined by qualitative GC) which represents a yield of 91.5%.

After crystallization from acetonitrile, a product of >98% purity was obtained. It contained 59.8% C and 30.3% Br (calc'd. 59.5% and 30.5%, respectively). Its mass spectrogram is given in Fig. 10.

## Example 13

### Preparation of 4,4'-bis(4"-bromo-2"-methylphenoxy)diphenyl ether (BBMPE)

Bromine (13.4 g, 0.084 moles) was added to a stirred solution of 4,4'-bis (2'-methylphenoxy) diphenyl ether (15.3 g, 0.04 moles) in dichloromethane (66 g) in a 100 ml three-necked round-bottomed flask equipped with a stirrer, a condenser, a dropping funnel and a thermometer. To the top of the condenser were attached two subsequent 20% NaOH traps to absorb hydrobromic acid released during the reaction. The addition of the bromine at a temperature in the range of 0-10 °C and in complete darkness took 120 minutes.

The progress of the reaction and its completion were determined by GC. Excess bromine and traces of hydrobromic acid were neutralized with aqueous 25% $NH_3$ (10 ml). The phases were separated and the organic layer washed with water (3 x 50 ml).

After distillation of the solvent in the organic phase, crude BBMPE was obtained (20.5 g), containing 94.1% of the desired isomer (determined by qualitative GC), which represents a yield of 89.3%. A purified sample was found to contain 57.9% C and 29.8% Br (calc'd. 57.8% and 29.6%, respectively). Its mass spectrogram is given in Fig. 15.

## Example 14

### Preparation of 4,4'-bis(4"-bromo-2"-methylphenoxy)diphenyl sulfone (BBMPS)

Bromine (13.4 g, 0.084 moles) was added to a stirred solution of 4,4'-bis (2"-methylphenoxy) diphenyl sulfone (17.2 g, 0.04 moles) in dichloromethane (66 g) in a 100 ml three-necked round-bottomed flask equipped with a stirrer, a condenser, a dropping funnel and a thermometer. To the top of the condenser were attached two subsequent 20% NaOH traps to absorb hydrobromic acid released during the reaction. The addition of the bromine at a temperature in the range of 0-10 °C and in complete darkness took 120 minutes.

The progress of the reaction and its completion were determined by GC. Excess bromine and traces of hydrobromic acid were neutralized with aqueous 25% $NH_3$ (10 ml). The phases were separated and the organic layer washed with water (3 x 50 ml).

After distillation of the solvent in the organic phase, crude BBMPS was obtained (22.0 g), containing 90% of the desired isomer (determined by qualitative GC) which represents a yield of 84.2%. A purified sample produced the mass spectrogram and NMR spectrum, attached as Figs. 11 and 12, respectively.

## Example 15

### Preparation of 2,2-bis [4'-(4"-bromo-2"-methylphenoxy)phenyl] propane (BBMPP)

Bromine (13.4 g, 0.084 moles) was added to a stirred solution of 2,2-bis [4'-(2"-methylphenoxy) phenyl] propane (16.3 g, 0.04 moles) in dichloromethane (50 ml) in a 100 ml three-necked round-bottomed flask equipped with a stirrer, a condenser, a dropping funnel and a thermometer. To the top of the condenser were attached two subsequent 20% NaOH traps to absorb HBr released during the reaction. The addition of the $Br_2$ at a temperature in the range 5-30 °C and in complete darkness took 120 minutes.

The progress of the reaction and its completion were determined by GC. Excess of bromine and traces of HBr were neutralized with aqueous 25% $NH_3$ (10 ml). The phases were separated and the organic layer washed with water (3 x 50 ml).

After distillation of the solvent in the organic phase, crude BBMPP was obtained (21.0 g), containing

90% of the desired isomer (determined by qualitative GC), which represents a yield of 83.5%.

Elemental analysis: 61.7% C and 28.1% Br, calc'd 61.5% and 28.3%, respectively.

**Example 16**

**Preparation of 4,4'-bis (4"-bromo-2"-methylphenoxy) diphenyl sulfide (BBMPSD)**

Bromine (13.4 g, 0.084 moles) was added to a stirred solution of 4,4'-bis (2"-methylphenoxy) diphenyl sulfide (15.9 g, 0.04 moles) in dichloromethane (50 ml) in a 100 ml three-necked round-bottomed flask equipped with a stirrer, a condenser, a dropping funnel and a thermometer. To the top of the condenser were attached two subsequent 20% NaOH traps to absorb HBr released during the reaction. The addition of the $Br_2$ at a temperature in the range 5-40 °C and in complete darkness took one hour, after which the reaction mixture was stirred for a further hour.

The progress of the reaction and its completion were determined by GC. Excess of bromine and traces of HBr were neutralized with aqueous 25% $NH_3$ (10 ml). The phases were separated and the organic layer washed with water (100 ml).

After distillation of the solvent in the organic phase, crude BBMPSD was obtained (21.5 g), containing 96% of the desired isomer (determined by qualitative GC), which represents a yield of 92.8%.

Elemental analysis: 55.9% C and 28.6% Br, calc'd 56.1% and 28.8%, respectively.

**Example 17**

**Preparation of 4,4'-bis(4"-bromo-2"-methylphenoxy) benzophenone (BBMPBP)**

Bromine (13.4 g, 0.084 moles) was added to a stirred solution of 4,4'-bis (2"-methylphenoxy) benzophenone (15.8 g, 0.04 moles) in dichloromethane (50 ml) in a 100 ml three-necked round-bottomed flask equipped with a stirrer, a condenser, a dropping funnel and a thermometer. To the top of the condenser were attached two subsequent 20% NaOH traps to absorb HBr released during the reaction. The addition of the $Br_2$ at a temperature in the range 5-30 °C and in complete darkness took one hour, after which the reaction mixture was stirred for a further hour.

The progress of the reaction and its completion were determined by GC. Excess of bromine and traces of HBr were neutralized with aqueous 25% $NH_3$ (10 ml). The phases were separated and the organic layer washed with water (100 ml).

After distillation of the solvent in the organic phase, crude BBMPBP was obtained (21 g), containing 95% of the desired isomer (determined by qualitative GC), which represents a yield of 90.4%.

Elemental analysis: 58.5% C and 28.8% Br, calc'd 58.7% and 29.0%, respectively.

**Example 18**

**Preparation of 4,4'-bis (4"-bromo-2"-methylphenoxy) diphenylmethane (BBMPD)**

Bromine (13.4 g, 0.084 moles) was added to a stirred solution of 4,4'-bis (2"-methylphenoxy) diphenylmethane (15.2 g, 0.04 moles) in dichloromethane (50 ml) in a 100 ml three-necked round-bottomed flask equipped with a stirrer, a condenser, a dropping funnel and a thermometer. To the top of the condenser were attached two subsequent 20% NaOH traps to absorb HBr released during the reaction. The addition of the $Br_2$ at a temperature in the range 5-30 °C and in complete darkness took one hour, after which the reaction mixture was stirred for a further hour.

The progress of the reaction and its completion were determined by GC. Excess of bromine and traces of HBr were neutralized with aqueous 25% $NH_3$ (10 ml). The phases were separated and the organic layer washed with water (100 ml).

After distillation of the solvent in the organic phase, crude BBMPD was obtained (19.8 g), containing 98% of the desired isomer (determined by qualitative GC), which represents a yield of 90.2%.

Elemental analysis: 59.9% C and 29.6% Br, calc'd 60.2% and 29.7%, respectively.

## Claims

**1.** A compound of the formula

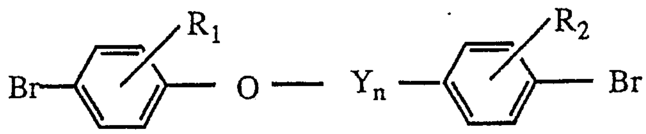

(I)

wherein:

n = 0 or 1;

$R_1$ and $R_2$ are $C_1$ to $C_3$ alkyl groups, or $R_2$ may be H when n = 0;

Y is selected from the group:

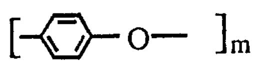 ; 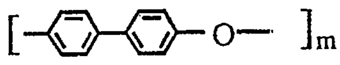

or

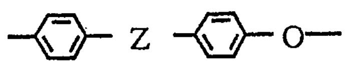

wherein:

m = 1-6;

Z = $SO_2$,S,C=O,$CH_2$ or $C(CH_3)_2$;

with the proviso that $R_1$ and $R_2$ cannot both be $CH_3$ when n = 0.

2. A process for preparing a compound of formula I, as defined in claim 1, comprising reacting a compound of formula II:

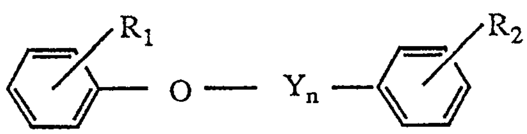

(II)

wherein $R_1$, $R_2$, Y and n have the meanings defined in claim 1 with a brominating agent in the substantial absence of light.

3. A process according to claim 2, wherein the reaction is carried out in the absence of solvent.

4. A process according to claim 2, wherein the bromination reaction is carried out in an organic solvent, which is substantially unreactive under the reaction conditions.

5. A process according to claim 4, wherein the organic solvent is a halocarbon or acetonitrile.

6. A process according to claim 5, wherein the halocarbon is selected from dichloromethane, chlorobenzene and 1,2-dichloromethane.

7. A process according to claim 2, wherein the reaction is carried out in a liquid medium comprising water and a halocarbon solvent.

8. A process according to any one of the preceding claims, wherein the bromination reaction is carried out at a temperature comprised between about -10° C and 90° C.

9. A process according to any one of the preceding claims, in which the brominating agent is selected from $Br_2$ and dibromodimethylhydantoin.

**10.** A process according to claim 9, wherein an oxidizing agent is added to the reaction mixture to liberate elemental bromine from HBr.

**11.** A process according to claim 10, wherein the oxidizing agent is selected from $H_2O_2$ and $NaBrO_3$.

**12.** A process according to claim 5, wherein the organic solvent is selected from the group consisting essentially of dichloromethane, 1,2-dichloroethane and acetonitrile.

**13.** 4,4'-Dibromo-3-methyl-diphenylether.

**14.** 4,4'-Dibromo-2-methyldiphenyl ether.

**15.** 4,4'-Dibromo-2-isopropyldiphenyl ether.

**16.** 1,4-Bis (4'-bromo-3'-methylphenoxy) benzene.

**17.** 1,4-Bis (4'-bromo-2'-methylphenoxy) benzene.

**18.** 4,4'-Bis (4"-bromo-2"-methylphenoxy) biphenyl.

**19.** 4,4'-Bis (4"-bromo-2"-methylphenoxy) diphenyl ether.

**20.** 4,4'-Bis (4"bromo-2"-methylphenoxy) diphenyl sulfone.

**21.** 4,4'-Bis (4"-bromo-2"-methylphenoxy) diphenylmethane.

**22.** 4,4'-Bis (4"-bromo-2"-methylphenoxy) diphenyl sulfide.

**23.** 2,2'-Bis [4'-(4"-bromo-2"-methylphenoxy) phenyl] propane.

**24.** 4,4'-Bis (4"bromo-2"-methylphenoxy) benzophenone.

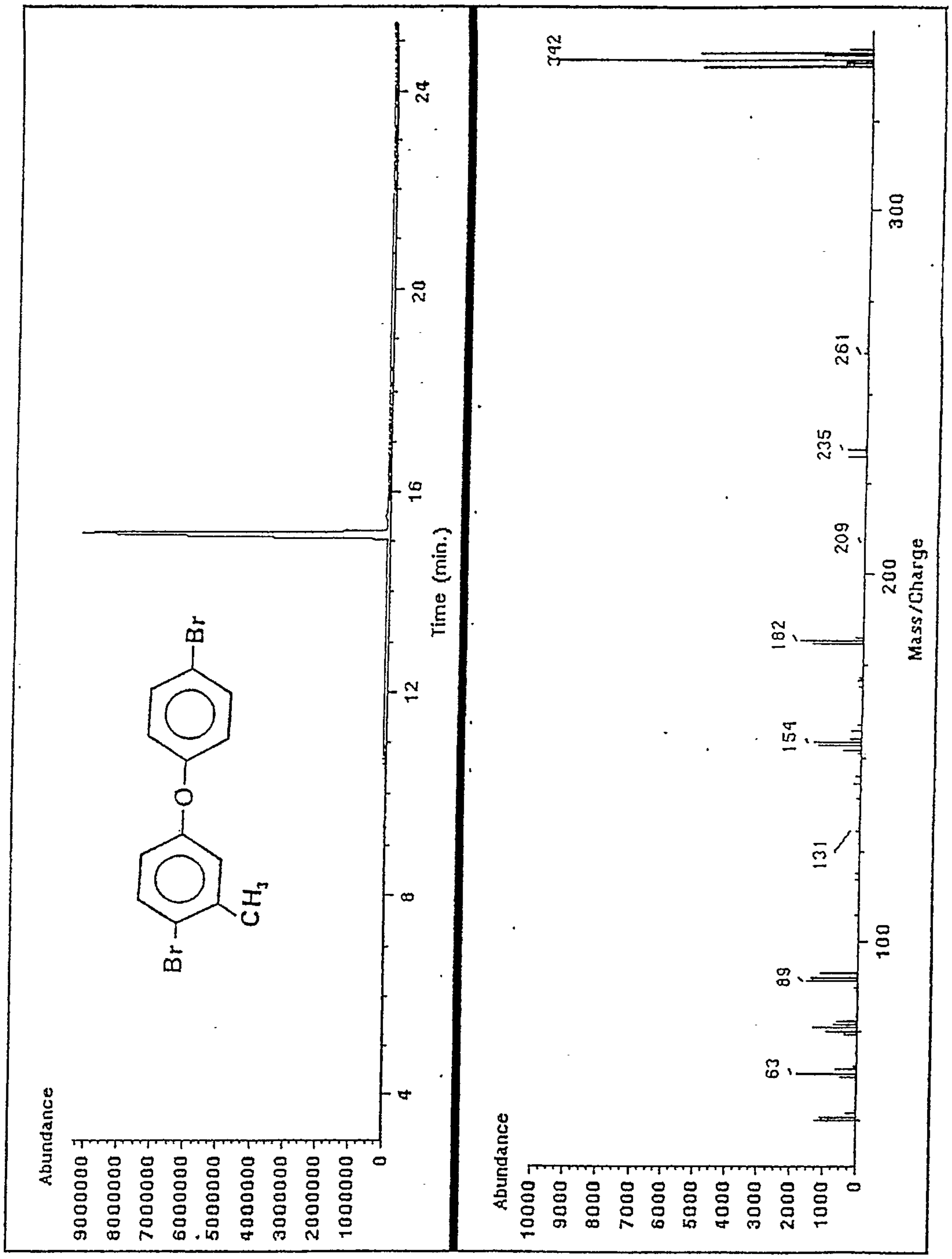
Abundance
9000000
8000000
7000000
6000000
5000000
4000000
3000000
2000000
1000000
0
4
8
12
16
20
24
Time (min.)
Abundance
10000
9000
8000
7000
6000
5000
4000
3000
2000
1000
0
63
89
100
131
154
182
200
209
235
261
300
342
Mass/Charge

Fig. 1

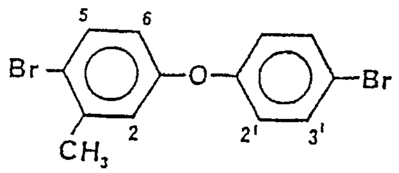

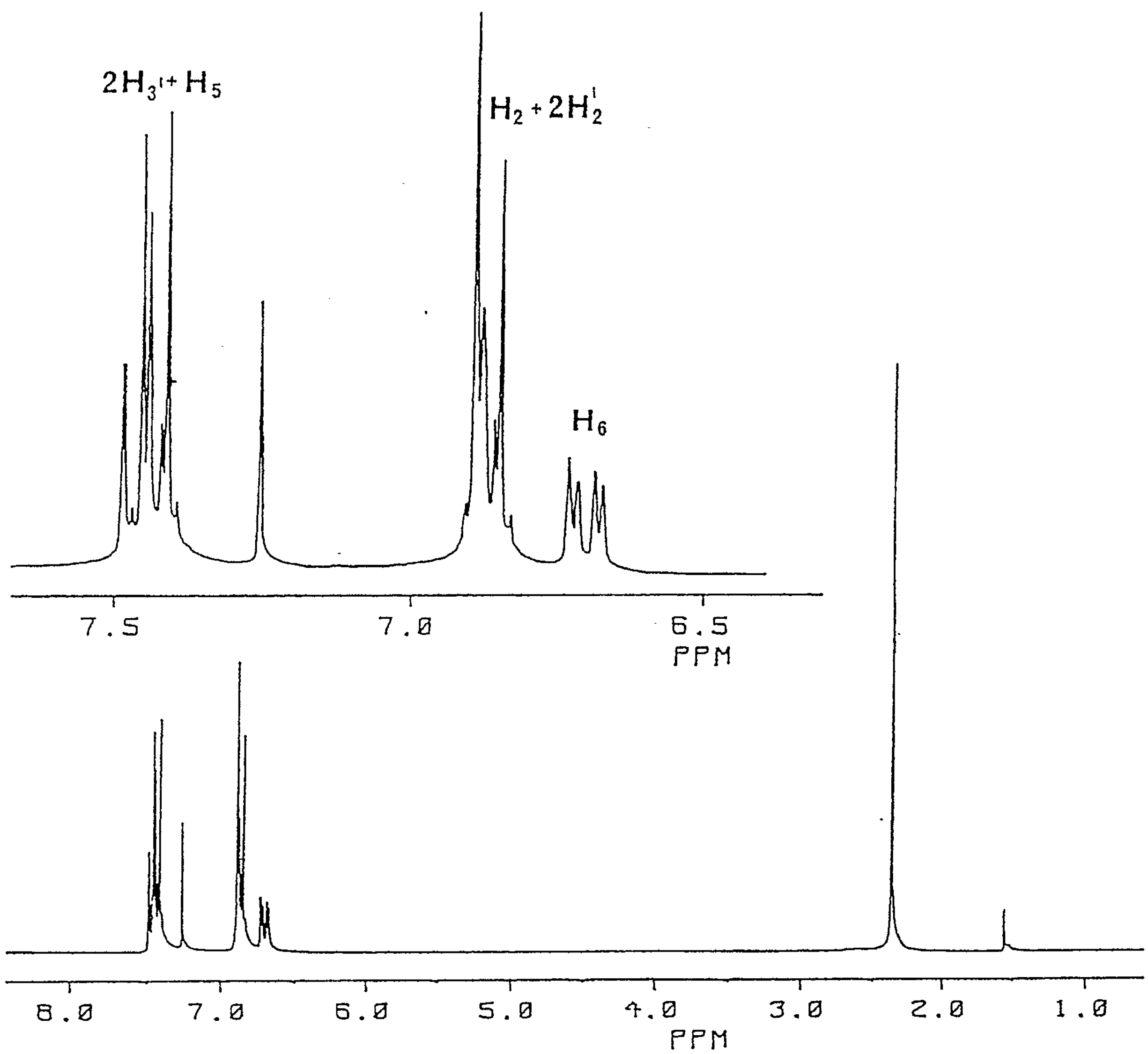


**Fig. 2**

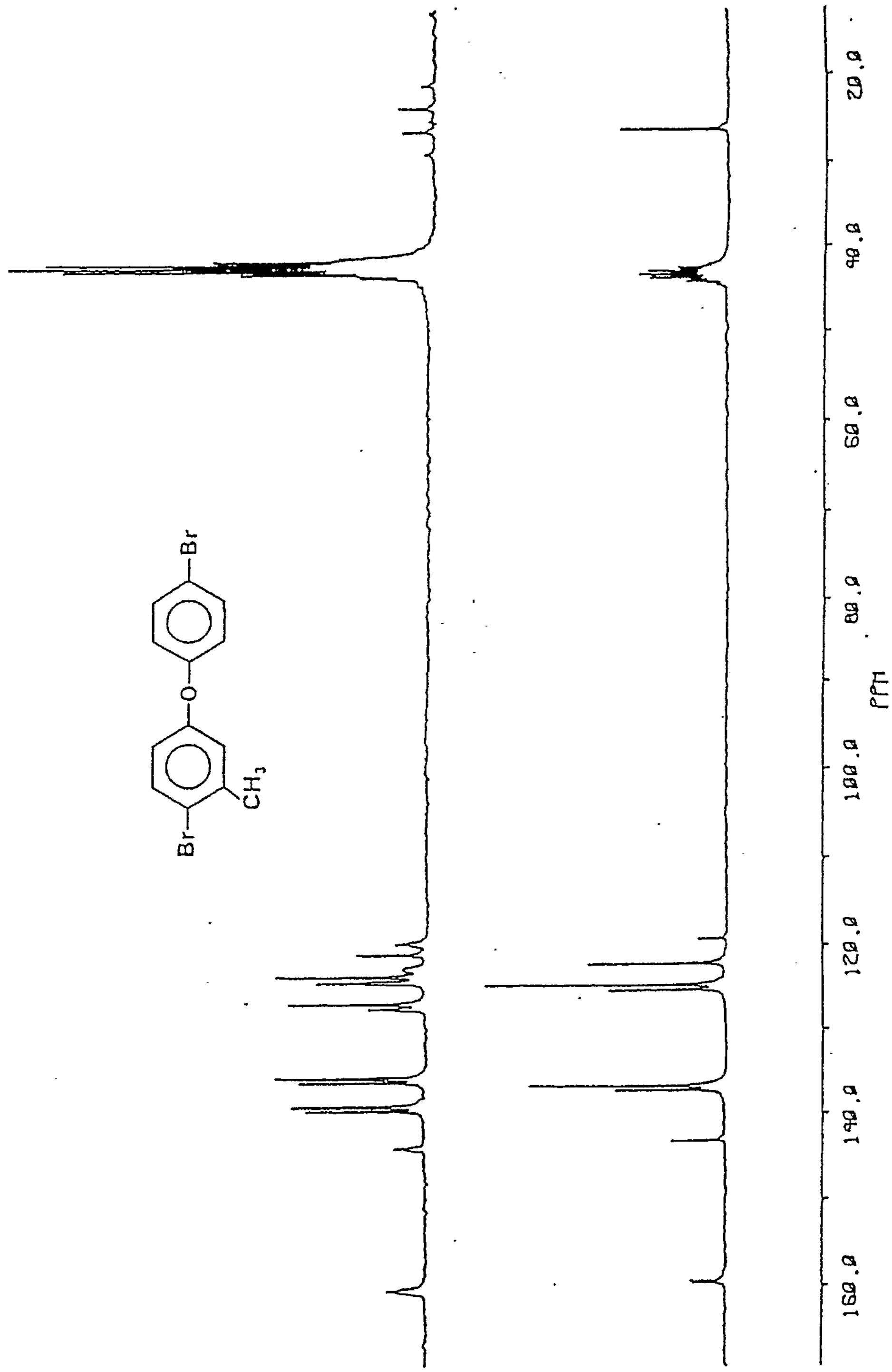


Fig. 3

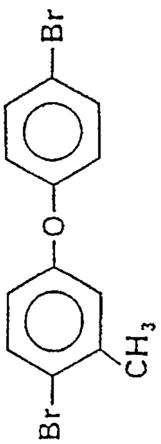

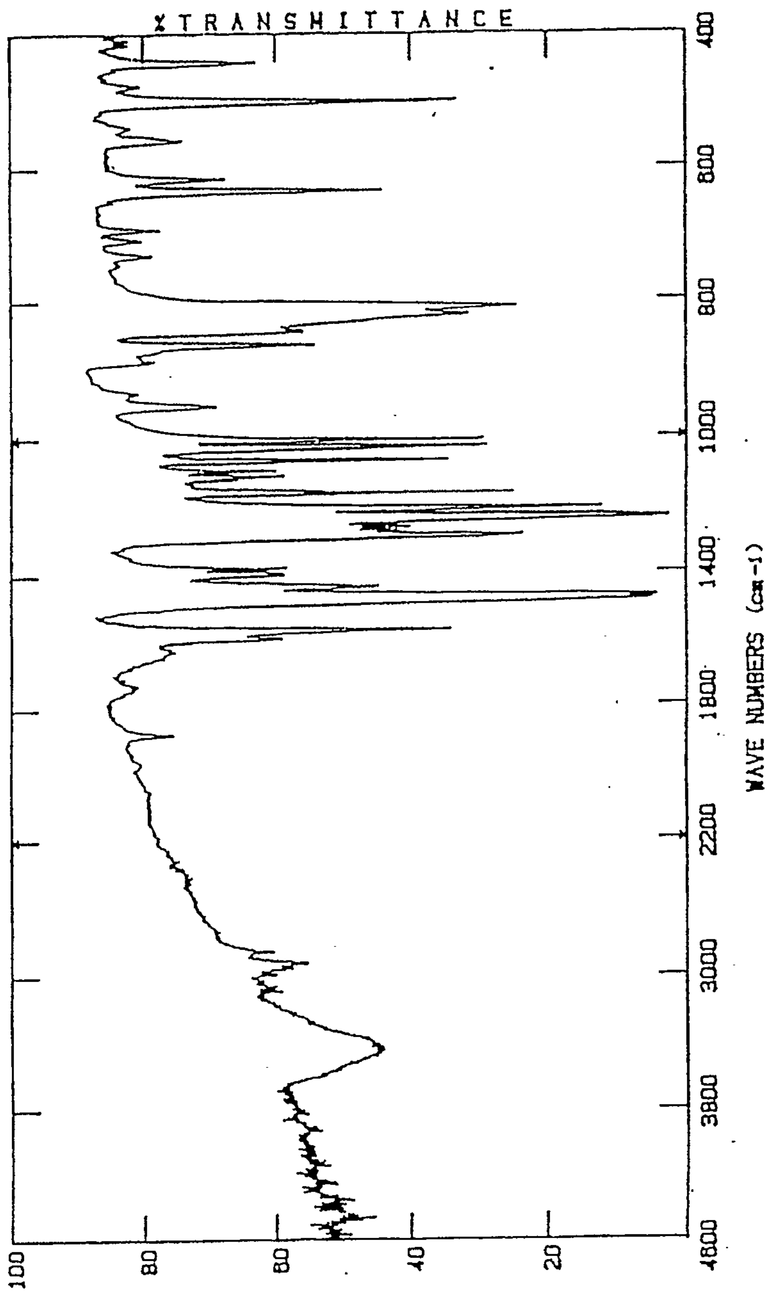


Fig. 4

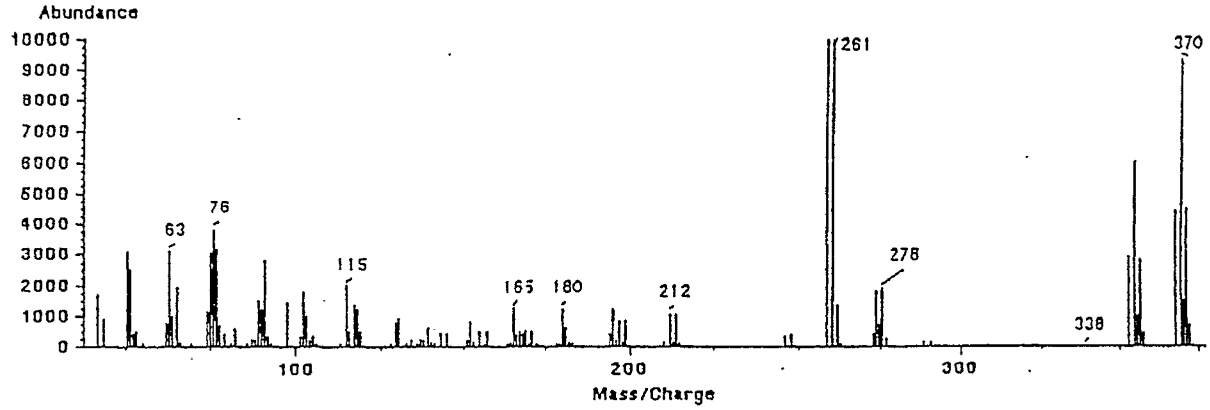
Abundance
10000
9000
8000
7000
6000
5000
4000
3000
2000
1000
0
63
76
115
165
180
212
261
278
338
370
100
200
300
Mass/Charge

Fig. 5

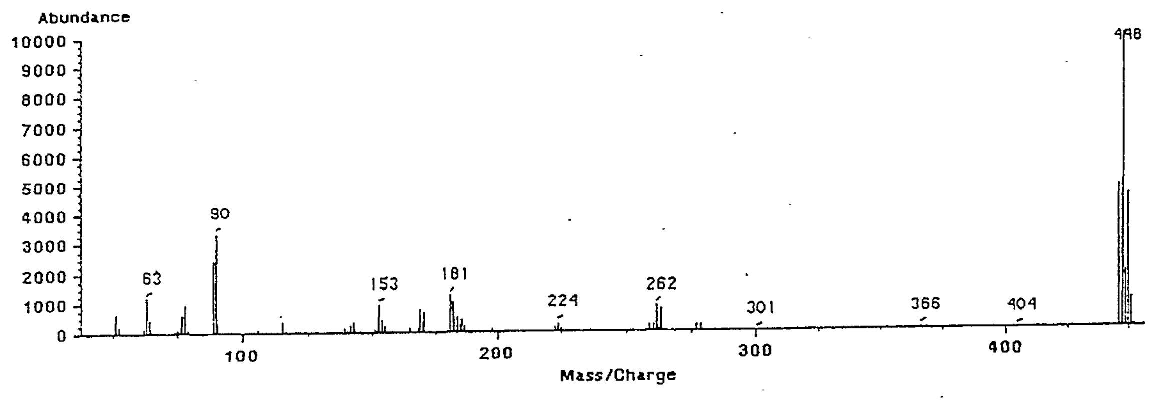
Abundance
10000
9000
8000
7000
6000
5000
4000
3000
2000
1000
0
63
90
153
181
224
262
301
366
404
100
200
300
400
Mass/Charge

Fig. 9

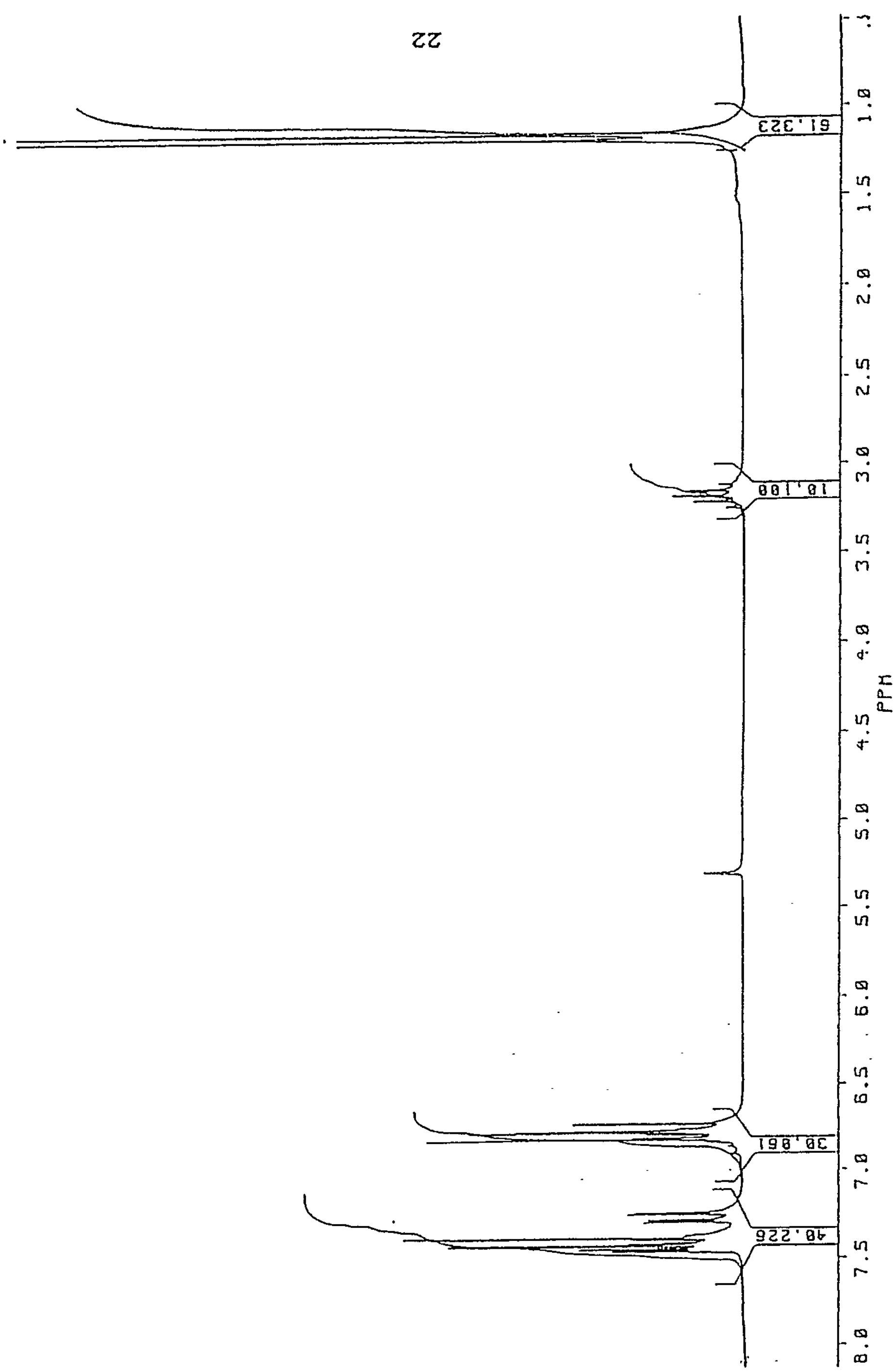
22
61.323
10.100
30.861
40.226
PPM
1.0
1.5
2.0
2.5
3.0
3.5
4.0
4.5
5.0
5.5
6.0
6.5
7.0
7.5
8.0

Fig. 6

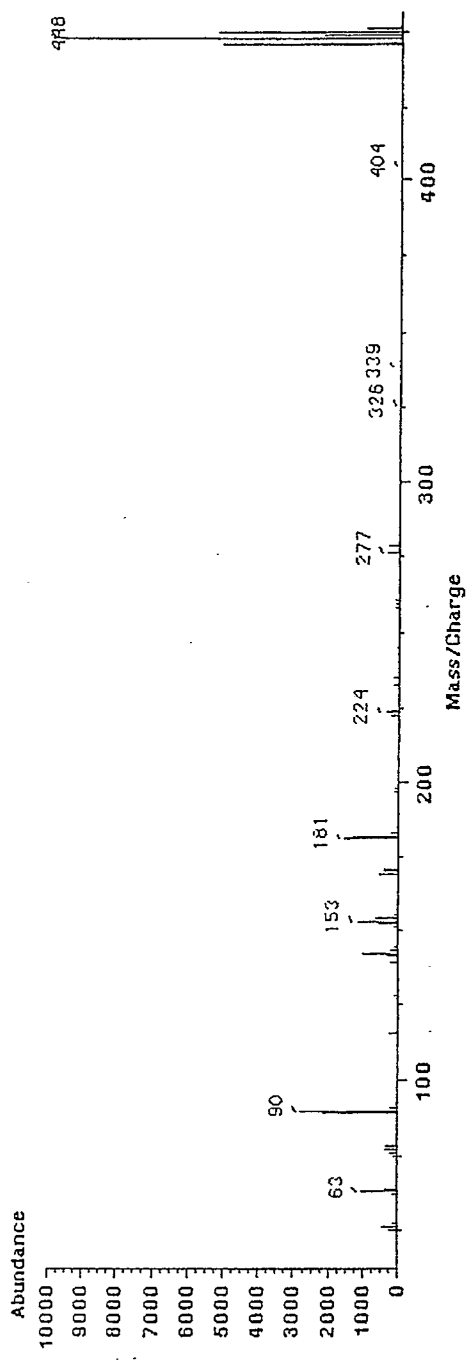
Abundance
10000
9000
8000
7000
6000
5000
4000
3000
2000
1000
0
63
90
153
181
224
277
326
339
404
488
100
200
300
400
Mass/Charge

Fig. 7

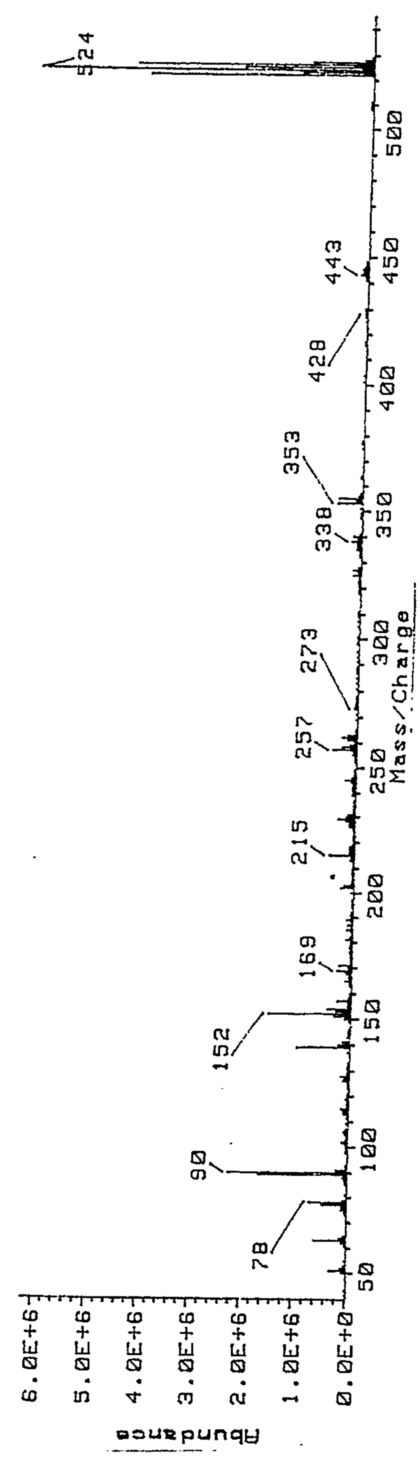
Abundance
6.0E+6
5.0E+6
4.0E+6
3.0E+6
2.0E+6
1.0E+6
0.0E+0
78
90
152
169
215
257
273
338
353
428
443
524
50
100
150
200
250
300
350
400
450
500
Mass/Charge

Fig. 10

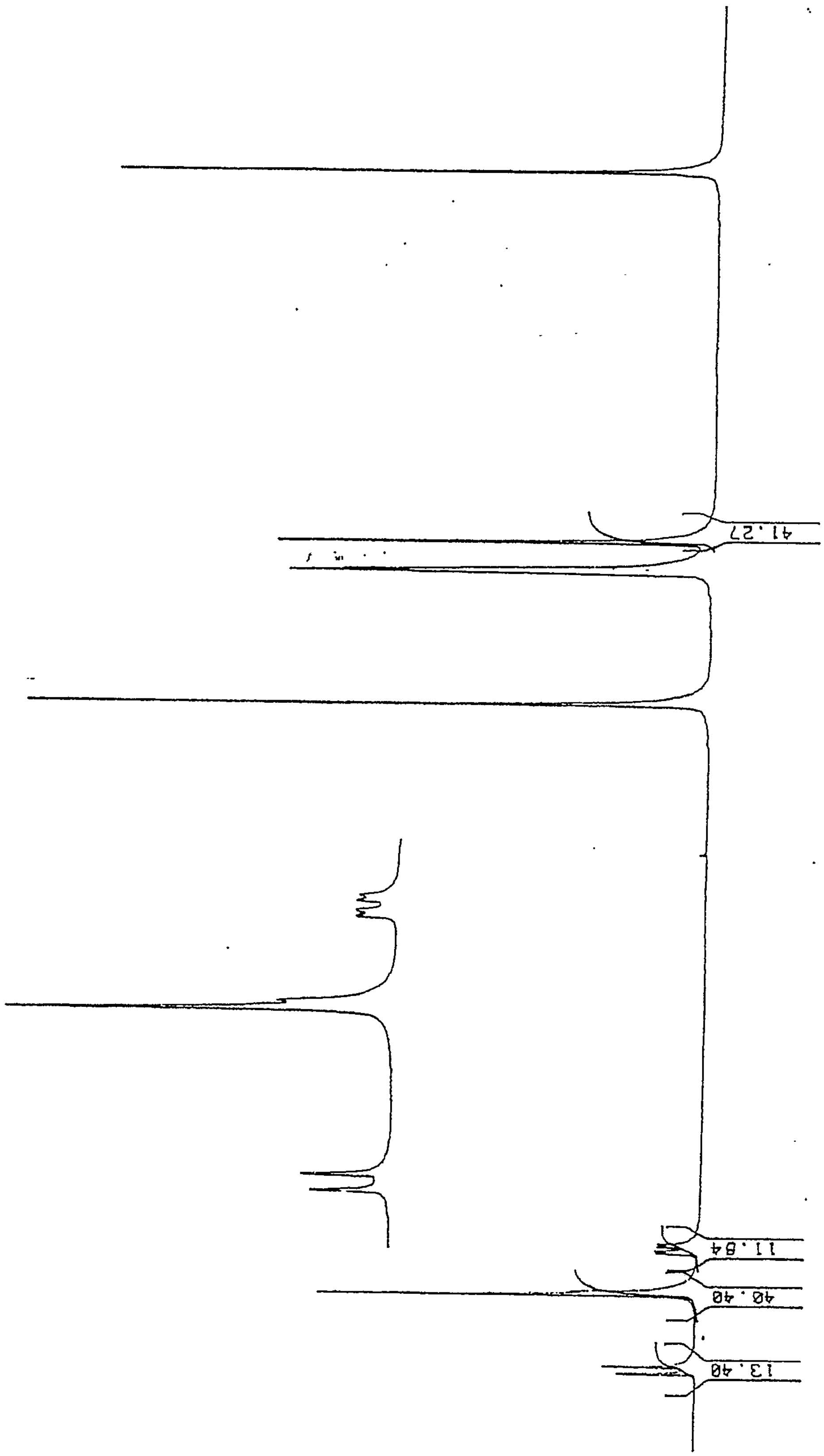


Fig. 8

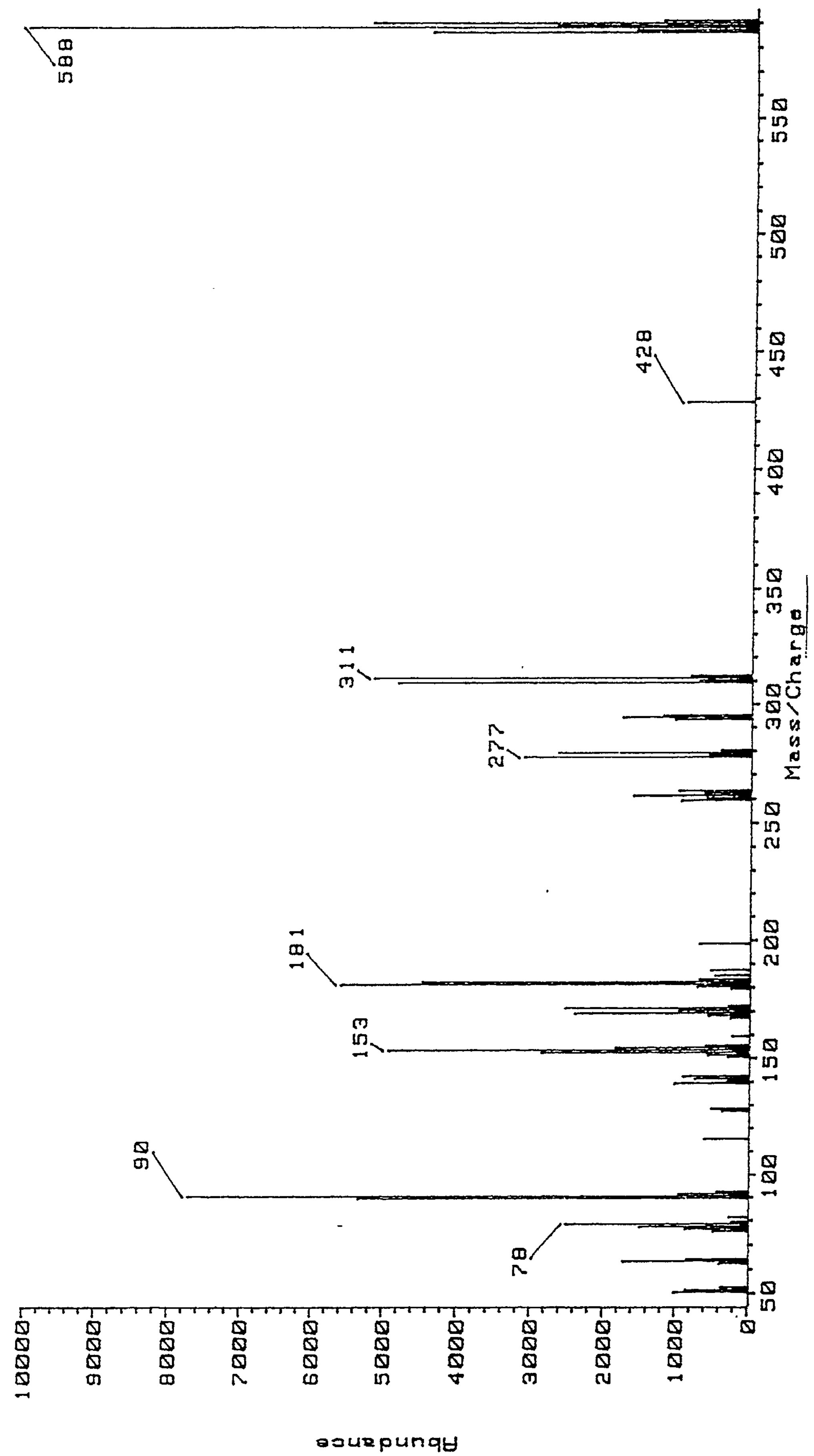
Abundance
10000
9000
8000
7000
6000
5000
4000
3000
2000
1000
0
Mass/Charge
50
100
150
200
250
300
350
400
450
500
550
78
90
153
181
277
311
428
588

Fig. 11

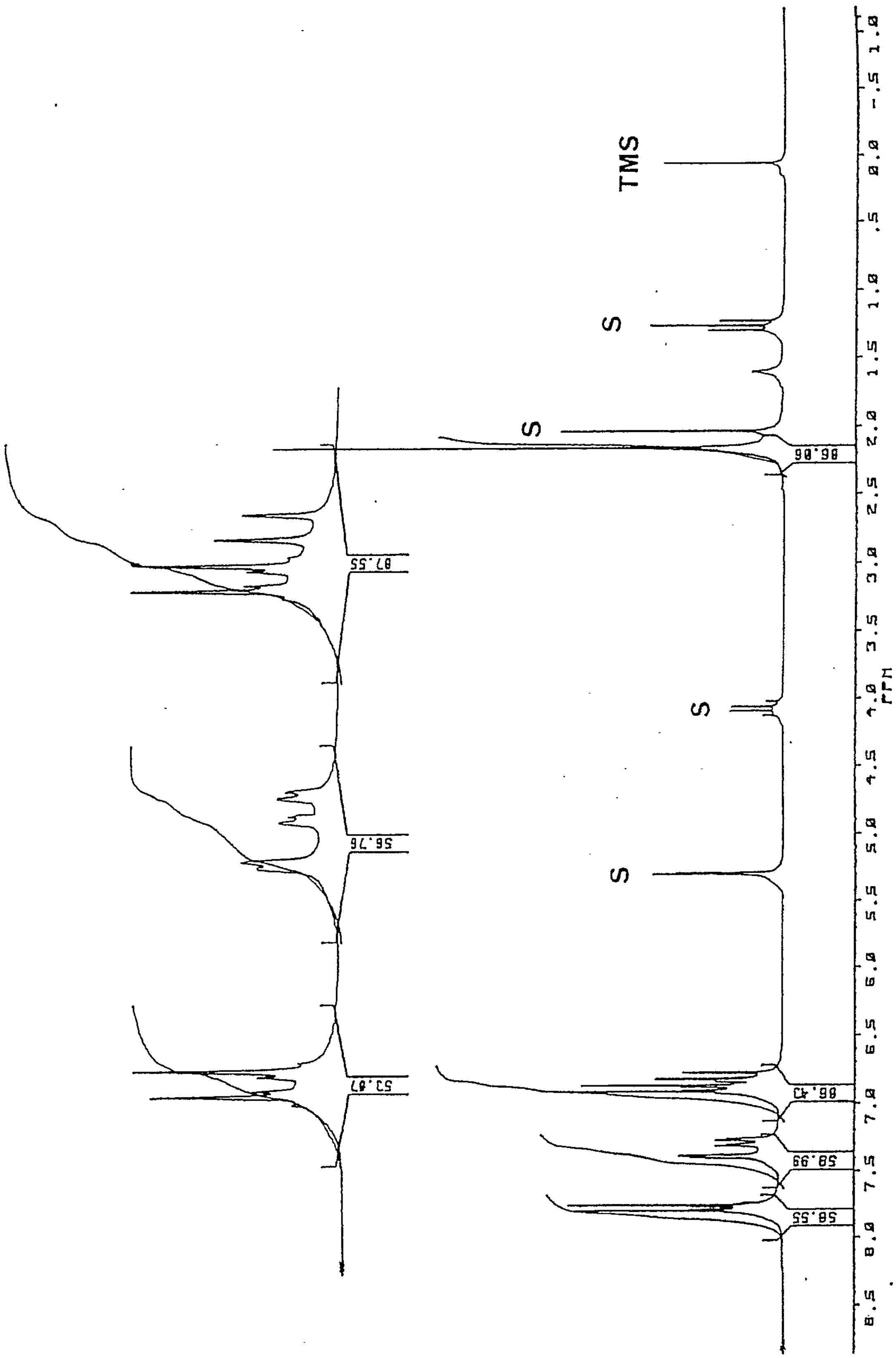


Fig. 12

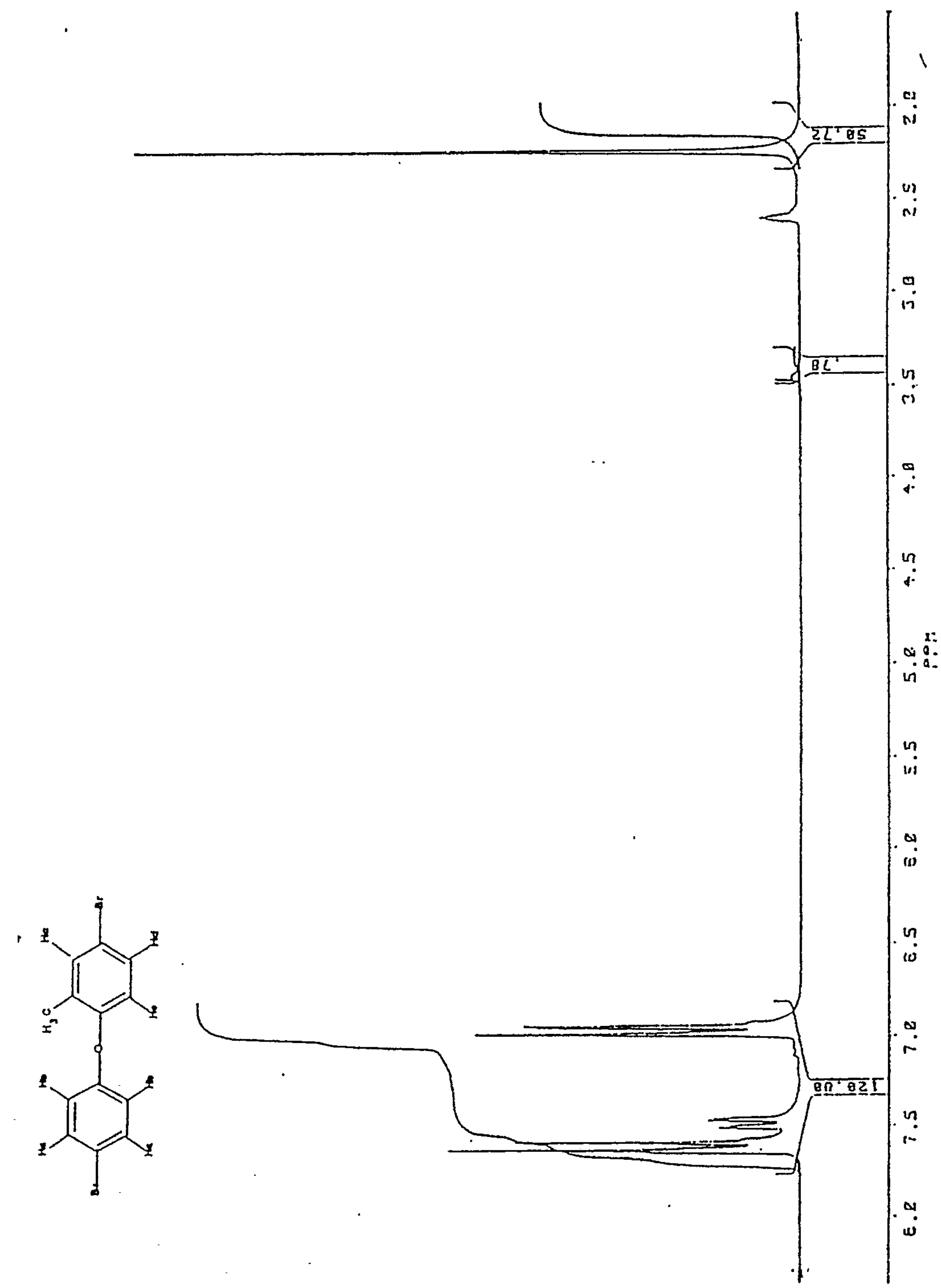


Fig. 14

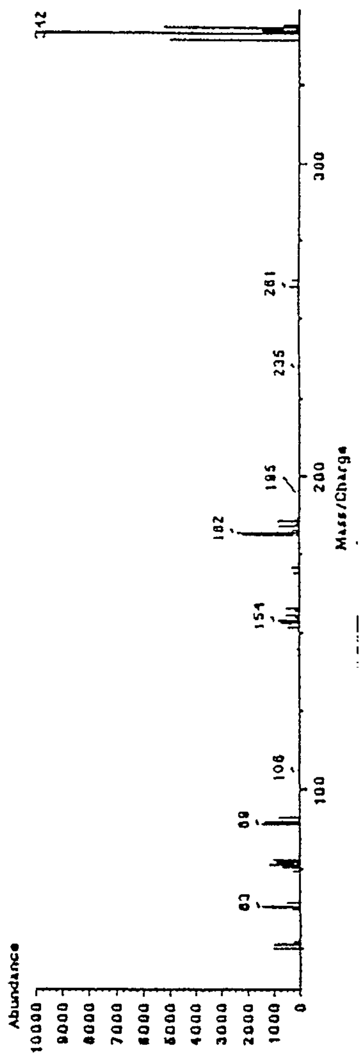
Abundance
10000
9000
8000
7000
6000
5000
4000
3000
2000
1000
0
63
89
106
154
182
195
235
281
312
100
200
300
Mass/Charge

Fig. 13

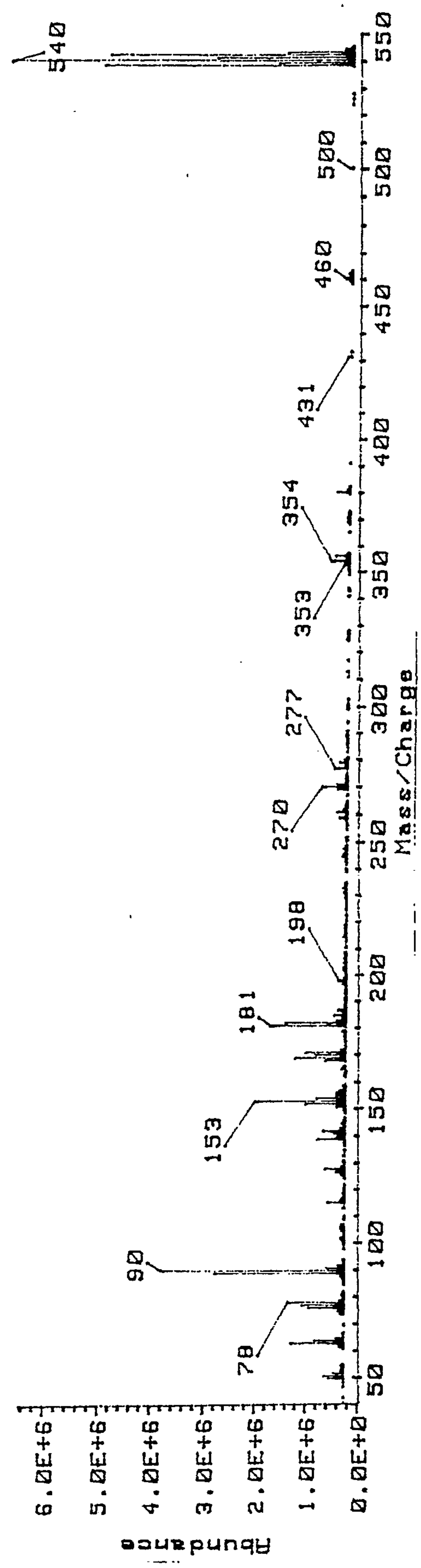
Abundance
6.0E+6
5.0E+6
4.0E+6
3.0E+6
2.0E+6
1.0E+6
0.0E+0
78
90
153
181
198
270
277
353
354
431
460
500
540
50
100
150
200
250
300
350
400
450
500
550
Mass/Charge

Fig. 15